# EUROPEAN PATENT SPECIFICATION

(11) **EP 4 504 028 B1**
(45) Date of publication and mention of the grant of the patent: **11.03.2026**
(21) Application number: 23718809.9
(22) Date of filing: 05.04.2023
(51) Int. Cl.: A61B 1/12, B65D 81/24, B65D 83/00, B65D 81/32, A61J 1/20

(54) **DETERGENT DELIVERY SYSTEM**
WASCHMITTELEINBRINGUNGSSYSTEM
SYSTÈME D'ADMINISTRATION DE DÉTERGENT

(30) Priority: 06.04.2022 GB 202205049
(43) Date of publication of application: 12.02.2025
(73) Proprietor: Meditech Endoscopy Limited, Tapton, Chesterfield S41 0TZ (GB)
(72) Inventor: RAMSEY, Peter, Tapton, Chesterfield S41 0TZ (GB); HAWKER, John, Hadleigh, Ipswich IP7 5FP (GB)
(74) Representative: Dummett Copp LLP
(86) International application number: PCT/GB2023/050912
(87) International publication number: WO 2023/194730

(56) References cited:
- US-A- 5 185 007
- US-A1- 2014 312 063
- US-A1- 2021 345 869

## Description

### FIELD OF THE INVENTION

The present invention relates to a detergent delivery system for use in cleaning an internal channel of a piece of equipment such as an endoscope or similar instrument. In particular this invention relates to a detergent delivery system that may be used to flush and clean the air/water channel of an endoscope. The invention also relates to a method of using the detergent delivery system to clean an air/water channel of an endoscope.

### BACKGROUND TO THE INVENTION

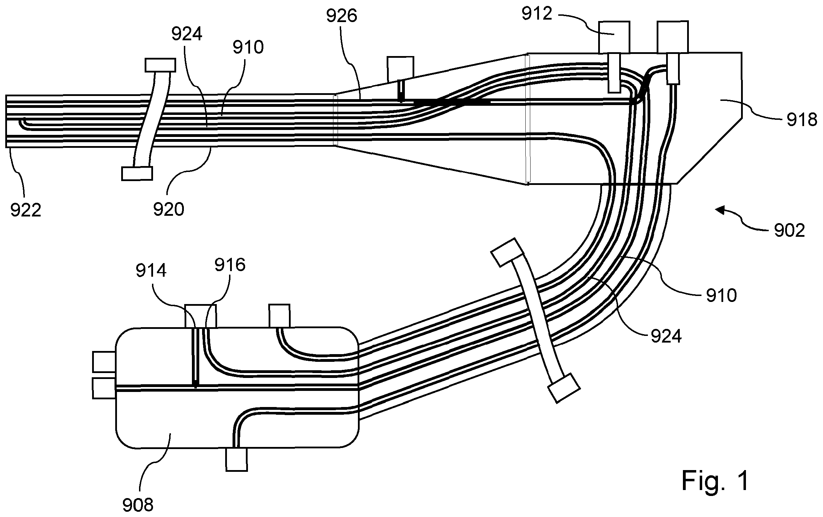

Figure 1 illustrates an air/water system of an endoscope 902. A first component of such an endoscope air/water system is an air pump in a light source. The air flows into the endoscope 902 through a light guide connector 908 of the endoscope 902. The air travels through a very small diameter channel 910 in the endoscope 902 and flows out of an air/water valve 912. The air/water system is controlled from a control section 918 of the endoscope 902. During use of the endoscope 902, in order to insufflate an organ to be examined, the air/water valve 912 is gently covered thereby preventing air exiting through the air/water valve 912 and instead diverting air down the insertion tube 920 and out of a distal tip 922 of the endoscope 902.

Another component of the air/water system is a water bottle. The water bottle is attached to air and water ports 914, 916 of the endoscope 902. During use, the air/water valve 912 may be depressed so that air entering the endoscope 902 is diverted through the air port 914 of the endoscope 902 and into the water bottle causing the bottle to be pressurized. This pressure then forces water from the bottle, through the water port 916, and into another small diameter channel 924 in the endoscope. The water travels along the channel 924 and may be used to flush or clear the view at the distal tip 922 of the endoscope 902.

The air/water channel is therefore actually formed by the two separate small diameter channels 910, 924 that combine into a single channel only proximate the distal tip 922 of the endoscope 902. The air/water channel 910, 924 has a significantly smaller diameter than a suction/biopsy channel 926 of the endoscope 902. Furthermore, a small metal nozzle is generally disposed at the tip 922 of the endoscope 902 to deflect air or water from the air/water channel 910, 924 across the image lens of the endoscope 902. The presence of this metal nozzle means that it is not possible to brush the air/water channel 910, 924. This, together with the small diameter of the air/water channel 910, 924, as well as other factors, makes it difficult to clean and decontaminate the air/water channel 910, 924.

The air/water nozzle is the area of greatest failure in the channel system of endoscopes as the air/water nozzle is the point of smallest internal diameter of the air/water channel system, which means that it can easily become blocked or obstructed by debris. Furthermore, during an endoscopic procedure, air infused into the organ being examined can force debris to back up into the nozzle and into the air/water channels 910, 924. This debris can travel some distance back into the channels.

After use of an endoscope, the endoscope must be cleaned and sterilised. Cleaning refers to the removal of visible debris, which may include both inorganic and organic substances. Cleaning usually involves a mechanical process, together with water and detergent. Following cleaning, sterilisation of the endoscope destroys microorganisms present on or in the endoscope. Sterilisation processes usually involve steam or a chemical sterilant. Following sterilisation, the endoscope undergoes high-level disinfection.

It is currently recommended that a preliminary cleaning routine should be undertaken immediately after use and before the endoscope is detached from the light source/video processor. Current pre-clean practice is to flush air and water through the air/water channel. It has been found, however, that routine pre-cleaning procedures for endoscopes do not remove biofilm reliably from endoscope channels. This leads to failures in the subsequent decontamination processes.

It is also recommended that the air and water channels (and any auxiliary channel) are also irrigated with detergent, not only to check for blockages but also to expel any blood, mucus and other debris. For example, document US 2021/345869 A1 discloses a detergent delivery system comprising a flexible bag for holding a liquid detergent, and a first and second fluid conduit extending from the inside to the outside of the bag adapted to be connected to water and air ports of an endoscope.

It is an object of the present invention to provide a cleaning apparatus that permits improved cleaning of the air/water channel of an endoscope, in particular with a detergent solution, and that overcomes at least one disadvantage of prior art endoscope channel flushing or cleaning systems whether referred to herein or otherwise.

### SUMMARY OF THE INVENTION

The present invention is defined in claim 1.

In preferred embodiments each of the first and second fluid conduits extend from a first end of the bag, and the first end of the first fluid conduit is at a first distance from a second end of the bag and the first end of the second fluid conduit is at a second distance from the second end of the bag, the second distance being different from the first distance.

One or both of the first and second fluid conduits may be flexible.

In some embodiments the reservoir comprises a sealed receptacle within the internal volume of the bag. The sealed receptacle may be rupturable upon pressure being applied to the receptacle by a user without the bag rupturing.

In other embodiments the reservoir is provided by a part of the first fluid conduit proximate the first end of the first fluid conduit. Preferably the first fluid conduit includes a channel extending along a length of said conduit for conveying fluid through said conduit, an end of the channel at the first end of the first fluid conduit is sealed, and the reservoir comprises a length of the channel proximate said sealed end. The sealed end of the first fluid conduit may be provided with a break-to-open feature. The break-to-open feature may comprise a tab that may be gripped by a user to remove the tab from the first fluid conduit.

In some embodiments a single cap element is attached to both the first end of the first fluid conduit and the first end of the second fluid conduit to block fluid flow through both the first and second fluid conduits. The single cap element is preferably separable from the first and second fluid conduits to allow fluid to flow through both of said conduits.

In preferred embodiments the second end of the first fluid conduit is connectable to an air port of an endoscope and the second end of the second fluid conduit is connectable to a water port of an endoscope.

The detergent delivery system may further comprise a connector attached to the second end of each of the first and second fluid conduits, the connector including an air port for connection to an air port of an endoscope, a water port for connection to a water port of an endoscope, a first channel fluidly connecting the first fluid conduit to the air port of the connector and a second channel fluidly connecting the second fluid conduit to the water port of the connector. The connector may be a rigid element. Preferably an axis of each of the air and water ports of the connector is perpendicular to an axis of each of the first and second fluid conduits.

In preferred embodiments the flexible bag contains a first volume of sterile water and the reservoir contains a second volume of detergent, the second volume being less than the first volume.

The invention further provides a method of cleaning an air/water channel of an endoscope using a detergent delivery system as claimed in claim 8.

In some embodiments the reservoir may be provided by a part of the first fluid conduit and the reservoir may be ruptured by providing a flow of air from the air port and into the first fluid conduit to increase the pressure in the reservoir. In yet further embodiments the reservoir may be provided by a part of the first fluid conduit and a single cap element may be attached to the first ends of both the first fluid conduit and the second fluid conduit to block fluid flow through both the first and second fluid conduits. In these embodiments the method preferably further comprises separating the cap element from the first and second fluid conduits so that, simultaneously, detergent flows from the first fluid conduit to mix with the sterile water and liquid is able to be drawn through the second fluid conduit to the water port of the endoscope.

### BRIEF DESCRIPTION OF THE DRAWINGS

The invention will now be further described, by way of example only, with reference to the accompanying drawings, in which:
Figure 1 illustrates a known air/water system of an endoscope;
Figure 2 shows a cleaning apparatus or detergent delivery system according to the present invention attached to an endoscope;
Figures 3 and 4 are perspective views of an example connector forming part of the cleaning apparatus of Figure 1 for attaching the cleaning apparatus to the endoscope;
Figure 5 shows a detergent delivery system not forming part of the present invention;
Figure 6 shows an embodiment of a detergent delivery system according to a first embodiment of the present invention;
Figure 7 shows an embodiment of a detergent delivery system according to a second embodiment of the present invention;
Figure 8 shows an embodiment of a detergent delivery system according to a second embodiment of the present invention;
Figure 9 shows an embodiment of a detergent delivery system according to a third embodiment of the present invention;
Figures 10a to 10c illustrate a break-off moulding forming part of the detergent delivery system of Figure 9;
Figures 11 to 15 show the detergent delivery system of Figure 9 with a solution containing pouch of the detergent delivery system removed;
Figure 16 shows an embodiment of a detergent delivery system according to a fourth embodiment of the present invention; and
Figures 17 to 20 illustrate a connector for a detergent delivery system for attaching the detergent delivery system to an endoscope.

### DESCRIPTION OF THE PREFERRED EMBODIMENTS

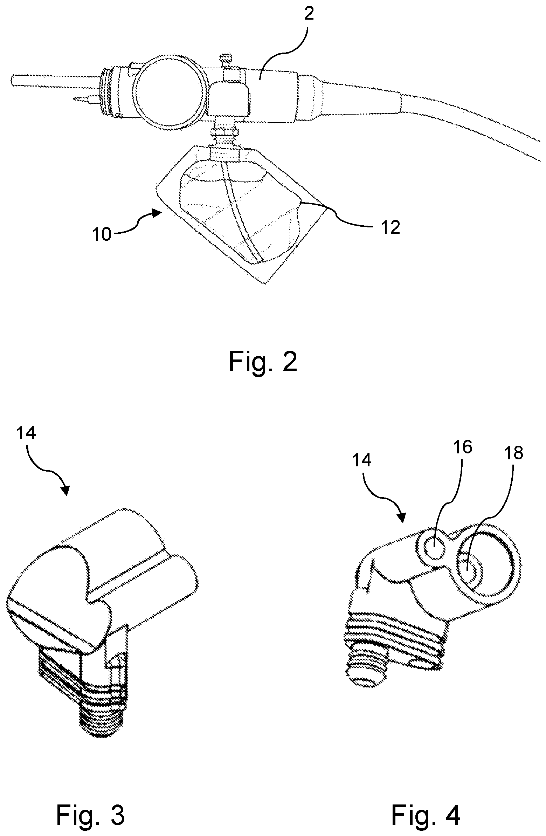

Figure 2 shows a detergent delivery system 10 including a bag or pouch 12 having an internal volume containing a detergent solution which may be connected to air and water ports of an endoscope 2. The pouch 12 is preferably flexible, and may be made of a sheet or film material. The detergent solution within the pouch 12 is preferably used to flush and clean the air/water channels of the endoscope 2 during a pre-clean procedure while the endoscope 2 is still attached to the light source.

The detergent delivery system 10 is preferably single use, and aims to standardise the pre-clean procedure. The detergent delivery system 10 is designed to be connected to the endoscope 2 and be supported solely by the endoscope 2. The detergent delivery system 10 will generally be suspended from the endoscope 2 during use. The size of the detergent delivery system 10 is therefore such that it holds sufficient detergent solution for a flush/clean of a single endoscope. The pouch 12 preferably holds up to 100 ml of detergent solution and may hold up to 150 ml or 200 ml of detergent solution.

Figures 3 and 4 illustrate a connector 14 that may be used to attach the pouch 12 to the air and water ports of the endoscope 2. The connector 14 is preferably configured such that the pouch 12 hangs substantially vertically from the connector 14. The connector 14 is preferably permanently attached to the pouch 12, for example by welding or a suitable adhesive.

The connector 14 includes an air port 16 for connection to the air port of the endoscope 2 and a water port 18 for connection to the water port of the endoscope 2. The air and water ports 16, 18 of the connector 14 may be covered by a suitable cover or sealing layer that is removed before use.

Because the detergent delivery system is designed to be single use, it is desirable if the detergent delivery system can be as simple and low cost as possible.

In one example, a detergent delivery system comprises a flexible pouch for holding a liquid and an elongate flexible conduit, a first end of the flexible conduit being disposed in an internal volume of the pouch and a second end of the flexible conduit being disposed in an internal volume of the pouch, and a central region of the flexible conduit being disposed external to the pouch and being bent or curved. Preferably the central region of the flexible conduit extends from a first or upper end of the pouch. Preferably the first end of the flexible conduit is at a first distance from a second or lower end of the pouch and the second end of the flexible conduit is at a second different distance from the second or lower end of the pouch.

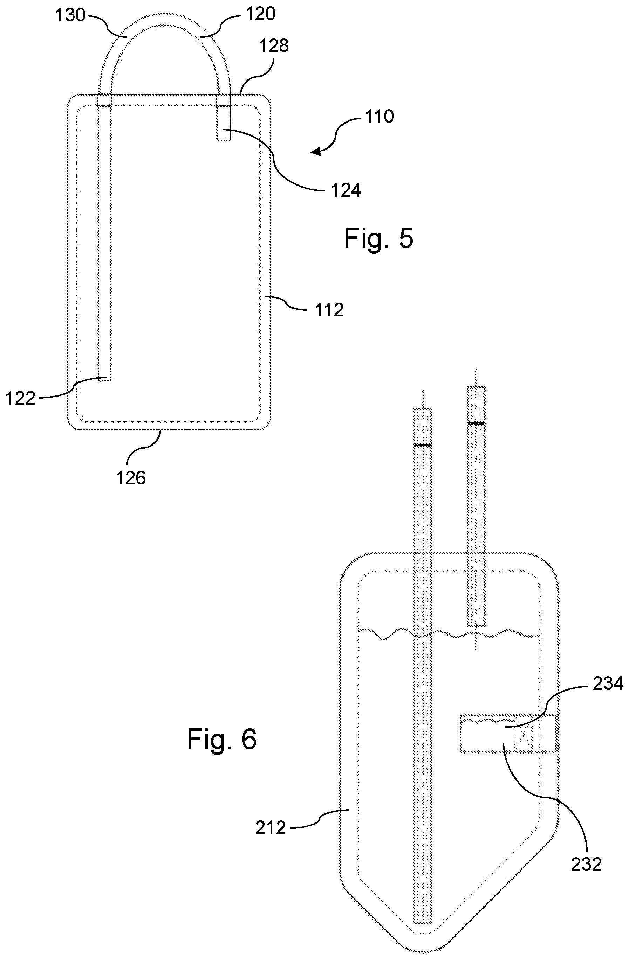

Figure 5 illustrates an example of such a detergent delivery system 110 including a pouch 112 and a fluid delivery conduit 120. The fluid delivery conduit 120 is elongate and extends between first and second ends 122, 124. The first end 122 is disposed within an internal volume of the pouch 112 proximate a lower end 126 of the pouch 112. The second end 124 is also disposed within the internal volume of the pouch 112 proximate an upper end 128 of the pouch 112. Although not illustrated in Figure 5, when the pouch 112 is filled with a detergent solution, the first end 122 of the fluid delivery conduit 120 is in the detergent solution and the second end 124 of the fluid delivery conduit 120 is preferably proximate or above the surface level of the detergent solution.

A part of the fluid delivery conduit 120, between the first and second ends 122, 124, extends out of the upper end 128 of the pouch 112. The fluid delivery conduit 120 is flexible and forms a bend or arc-shaped portion 130 that protrudes from the pouch 112. The fluid delivery conduit 120 may be in the form of a suitable polymeric tube.

When a user wishes to connect the detergent delivery system 110 to an endoscope, the fluid delivery conduit 120 is cut in the portion of the fluid delivery conduit 120 external to the pouch 112 (i.e. within the arc-shaped portion 130). The cut ends of the fluid delivery conduit 120 may then be connected to the air and water ports of the endoscope. In particular, after being cut, a first liquid conduit is formed between the first end 122 of the fluid delivery conduit 120 and a first cut end, and a second air conduit is formed between the second end 124 of the fluid delivery conduit 120 and a second cut end. Accordingly, the first cut end is attached to the water port of the endoscope and the second cut end is attached to the air port of the endoscope.

The pouch 112 preferably contains a neutral pH enzymatic detergent solution. The detergent is designed to break down proteinaceous materials and prevent the formation of biofilm in the air/water channel of the endoscope.

The detergent solution in the pouch is preferably of a relatively low concentration. It has been found, however, that pre-mixed low concentration detergent degrades over time. Tests show that bacterial growth occurs in low concentration detergent. Accordingly, it is desirable if the low concentration detergent solution is used as soon as it is formed. One option is to separate, within the pouch, a volume of sterile water and a volume of concentrated detergent. These two volumes could then be mixed to create the desired low concentration detergent solution shortly before using the solution to flush and clean the endoscope.

In preferred examples, therefore, a detergent delivery system comprises a flexible pouch for holding a liquid and a reservoir for holding a volume of concentrated detergent within an internal volume of the pouch. A first fluid conduit extends between a first end within the internal volume of the pouch and a second end external to the pouch, and a second fluid conduit extends between a first end within the internal volume of the pouch and a second end external to the pouch. The reservoir may be provided at the first end of one of the first and second fluid conduits. The reservoir may be provided by a channel or bore of one of the first and second fluid conduits. The reservoir may be rupturable by application of pressure to the reservoir to allow dispensing of the concentrated detergent from the reservoir into the internal volume of the pouch. The reservoir may be provided with a break-to-open feature to allow dispensing of the concentrated detergent from the reservoir into the internal volume of the pouch.

Figures 6 to 16 illustrate examples of detergent delivery systems that include a detergent concentrate reservoir. In these systems the internal volume of the pouch is initially filled with sterile water. The detergent concentrate reservoir is filled with a detergent concentrate. When a user wishes to utilise the detergent delivery system, and preferably after the detergent delivery system has been fitted to the endoscope, the detergent concentrate reservoir is designed to be ruptured or opened in some manner to dispense the detergent concentrate into the sterile water. The detergent and water can then be mixed to form the required relatively low concentration detergent solution. Preferably the reservoir is designed to be ruptured after the detergent delivery system is attached to the air and water ports of the endoscope.

In a first example, shown in Figure 6, a detergent concentrate reservoir 232 is a receptacle in the form of a sachet or sealed tube 234 disposed within the internal volume of the pouch 212. As in the illustrated example, the reservoir sachet 234 may be attached to an edge or side seam of the pouch 212. The sachet 234 may be positioned with respect to the pouch 212 such that, once the reservoir sachet 234 has been filled with detergent and the pouch 212 has been filled with water, the sachet 234 may be sealed concurrently with the pouch 212.

The detergent concentrate reservoir 232 is preferably disposed below the surface of the sterile water within the pouch 212 when the internal volume of the pouch 212 is filled with sterile water.

In some examples the sachet 234 is designed such that the sachet ruptures when pressure is applied to it. Accordingly, the material from which the sachet is formed may be thinner than the material from which the pouch is formed. Alternatively, the sachet may have a break-to-open feature.

In other examples the reservoir may be in the form of a separate small sachet that can be ruptured when pressure is applied to it. The sachet may be separate, i.e. not attached to the sides of the pouch, but retained within the internal volume of the pouch. The detergent concentrate reservoir could be in the form of a spherical 'bead' that is ruptured when it is squeezed.

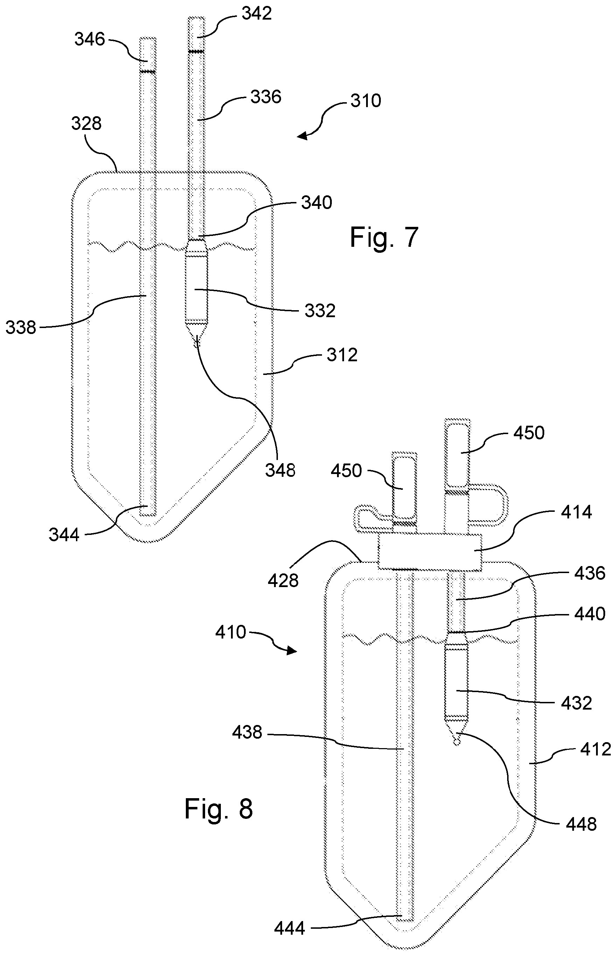

Figures 7 and 8 show examples of a detergent delivery system 310, 410 in which the detergent concentrate reservoir 332, 432 is disposed at an end of an air inlet conduit 336, 436. In these examples, the detergent delivery system 310, 410 comprises a pouch 312, 412 having an upper edge 328, 428. The air inlet conduit 336, 436 extends though the upper edge 328, 428 of the pouch 312, 412 such that a first end 340, 440 of the air inlet conduit 336, 436 is within an internal volume of the pouch 312, 412. The air inlet conduit 336, 436 extends from the upper edge 328, 428 of the pouch 312, 412 such that a second end of the air inlet conduit 336, 436 is external to the pouch 312, 412. Similarly, the detergent delivery system 310, 410 comprises a liquid outlet conduit 338, 438 that extends though the upper edge of the pouch 312, 412 such that a first end 344, 444 of the liquid outlet conduit 338, 438 is within an internal volume of the pouch 312, 412. The liquid outlet conduit 338, 438 extends from the upper edge 328, 428 of the pouch 312, 412 such that a second end of the liquid outlet conduit 338, 438 is external to the pouch 312, 412.

In the example of Figure 7, a second end 342, 346 of each of the air inlet conduit 336 and liquid outlet conduit 338 is configured to attach directly to each of the air port and water port of the endoscope respectively. To prevent liquid leaking from the conduits before use, the second ends 342, 346 of the conduits are preferably sealed, for example by heat sealing. When a user wishes to use the detergent delivery system, the user preferably cuts or otherwise breaks the sealed ends of the conduits before attaching the conduits to the ports of the endoscope. In other examples, the second end of each of the air inlet conduit and the liquid outlet conduit may be sealed by any suitable means. The second end of each of the air inlet conduit and the liquid outlet conduit may be sealed by disposable clamps.

The detergent concentrate reservoir 332, 432 is disposed at the first end 340, 440 of the air inlet conduit 336, 436. In these examples (Figures 7 and 8), the detergent concentrate reservoir 332, 432 is in the form of an enlarged region of conduit, i.e. having an internal and/or external diameter greater than the respective internal and/or external diameter of the rest of the air inlet conduit 336, 436. An end of the detergent concentrate reservoir 332, 432 includes a break-to-open feature 348, 448.

The detergent concentrate reservoir 332, 432 may be in the form of a cylindrical chamber or tube with a break-to-open end cap moulding. In use, a user grasps a part of the end cap and ruptures it or tears it from the air inlet conduit, thereby releasing detergent concentrate held within the reservoir into the sterile water within the pouch.

It will be appreciated that in these examples, the closing of the first end 340, 440 of the air inlet conduit 336, 436 by the detergent concentrate reservoir 332, 432 and break-to-open feature 348, 448 also means that it will not be possible for the endoscope to blow air into the bag or pouch 312, 412 to pressurise it until the end of the detergent concentrate reservoir 332, 432 or chamber has been opened. Accordingly, this design of reservoir provides a fail-safe solution that ensures that the pouch can only be pressurised by air entering the pouch, and liquid can only flow out of the pouch, if the detergent concentrate chamber has been opened or ruptured.

In some embodiments, the break-to-open feature at the end of the conduit used to seal the reservoir may be configured such that sufficient pressure within the air inlet conduit ruptures or otherwise breaks a part of the break-to-open feature. In these embodiments, in use, the detergent delivery system may be connected to the air/water ports of an endoscope and the control section of the endoscope may be used to divert air out of the endoscope air port. The air will enter the air inlet conduit but, because the reservoir at the end of the conduit is initially sealed, this will cause pressure in the air inlet conduit to increase. When the pressure reaches a threshold pressure this, preferably, causes the break-to-open feature to rupture and forces the detergent into the sterile water. Continued activation of the endoscope will then pressurise the pouch and force detergent solution through the liquid outlet conduit and into the air/water channels of the endoscope.

As shown in the example in Figure 8, rather than the second ends of the conduits being sealed, as in Figure 7, the second end of each of the air inlet conduit 436 and liquid outlet conduit 438 may be connected to a cap or connector 414. The connector 414 may include an air port for attaching to the air port of an endoscope and a liquid port for attaching to a water port of an endoscope. The air inlet conduit 436 is preferably fluidly connected to the air port of the connector 414 and the liquid outlet conduit 438 is preferably fluidly connected to the liquid port of the connector 414. Each of the air port and liquid port of the connector 414 may include a break-off tip or cap 450. A user preferably breaks off the tips or caps 450 before the connector 414 is attached to the ports of the endoscope. The tips or caps 450 are preferably single use so that they cannot be reattached to the air port and liquid port of the connector 414. The tips or caps 450 may remain connected to the connector 414, even after they have been broken from the ports, by sprues, cords or straps. This prevents the small caps or tips 450 being dropped or lost, and allows them to be disposed of or recycled together with the rest of the detergent delivery system 410.

The detergent concentrate reservoir 232, 332, 432 (of any of the above examples) may contain about 1 ml of detergent concentrate. The pouch 212, 312, 412 may contain about 30 ml of sterile water. As described above, prior to, or preferably after, fitting the pouch to an endoscope the reservoir is punctured or ruptured to release the detergent concentrate into the sterile water where it will be mixed to provide a low concentration detergent solution as the fluid is pumped out of the pouch into the air/water channel of the endoscope.

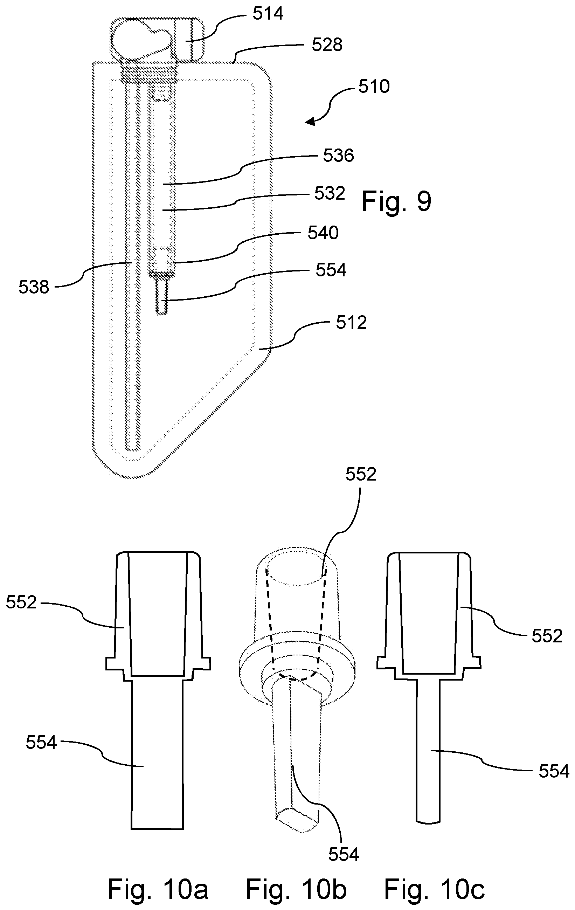

Figures 9 to 15 illustrate a further example of a detergent delivery system 510. In this example, a break-off moulding 552 including a flat blade tab 554 is disposed at the first end 540 of the air inlet conduit 536. This flat blade tab 554 is easy to grip with a user's thumb and finger from both sides of the pouch 512, allowing the user to grip the tab 554 and break it off from the end of the conduit 536.

The air inlet conduit 536 may provide a detergent concentrate reservoir 532. As such, the removal of the tab 554 from the end of the air inlet tube 536 both permits detergent concentrate to exit the reservoir and mix with the water within the pouch 512 and allows air to enter the pouch to pressurise the pouch when the detergent delivery system 510 is connected to the air/water ports of an endoscope.

In this example, the air inlet conduit 536 and the liquid outlet conduit 538 are attached to a connector 514 that is heat sealed to the pouch 512 at an upper edge 528 of the pouch 512. The connector 514 includes two ports 516, 518 that are configured to attach to the air/water ports of an endoscope. The connector 514 is arranged such that an axis of each of the ports of the connector is generally transverse or perpendicular to an axis of each of the air inlet conduit 536 and liquid outlet conduit 538. This allows the pouch 512 to hang substantially vertically when the detergent delivery system 510 is connected to an endoscope.

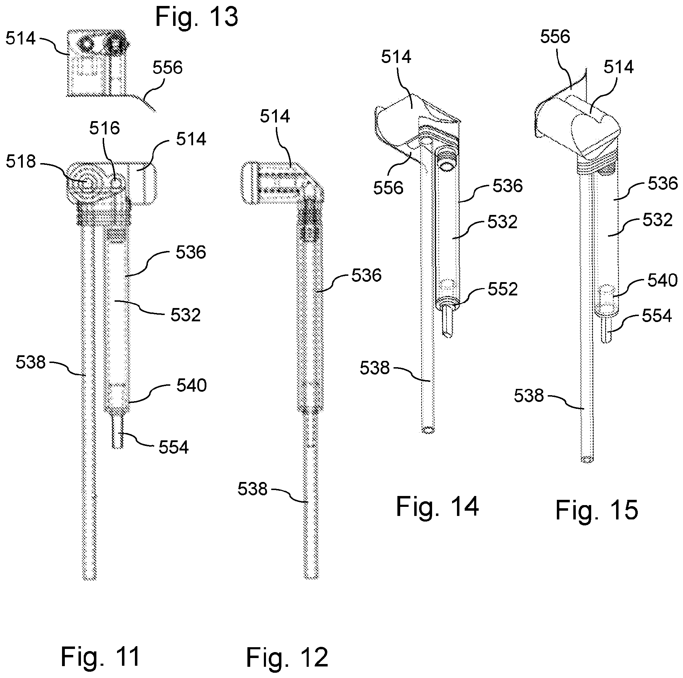

Figures 13, 14 and 15 show a cover or sealing layer 556 that seals the air and water ports 516, 518 of the connector 514 before use, and which is removed by a user when the connector 514 is to be attached to an endoscope.

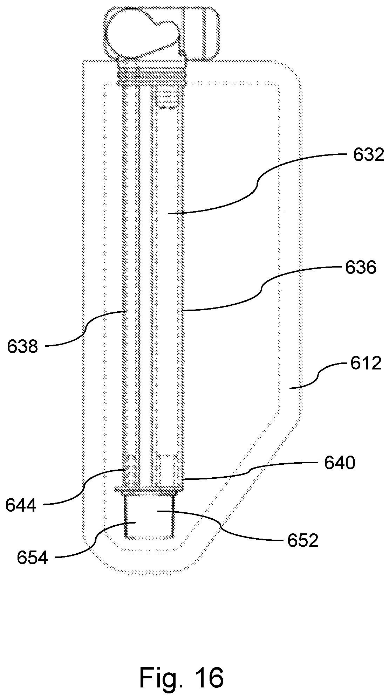

Figure 16 shows a further example in which a single break-off moulding 652 is disposed at the first ends 640, 644 of both of the air inlet conduit 636 and the liquid outlet conduit 638. This combined break-off moulding 652 provides a cross contamination protection for the contents of the detergent reservoir 632 (in the air inlet conduit 636) and the sterile water inside the bag or pouch 612. Breaking off the tab 654 on the combined moulding 652 opens the air inlet conduit 636 and detergent concentrate reservoir 632, allowing air in to pressurise the pouch 612 and releasing detergent concentrate into the sterile water. It also opens the liquid outlet conduit 638 which allows the detergent solution to exit the pouch 612 and enter the endoscope.

In one example a detergent delivery system comprises a flexible pouch for holding a liquid, a first fluid conduit extending between a first end within the internal volume of the pouch and a second end external to the pouch, a second fluid conduit extending between a first end within the internal volume of the pouch and a second end external to the pouch, and a connector attached to the second ends of the first and second fluid conduits, the connector comprising air and water ports for attachment to air and water ports of an endoscope, and at least one of the air and water ports of the connector including a one-way valve arranged to prevent the flow of fluid from the pouch out of the connector when the valve is closed. Preferably the valve is a reed valve or duck bill valve.

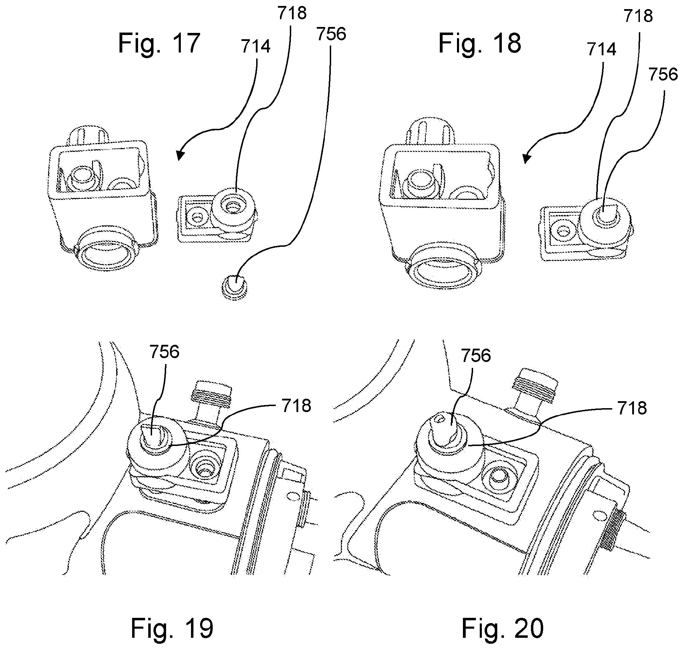

Figures 17 to 20 illustrate a connector 714 for a detergent delivery system including reed valves or duck bill valves 756. This connector 714 may, for example, be similar to the connector shown in Figures 11 to 15.

The reed valves 756 are preferably fitted to the liquid outlet port 718 of the connector 714. Miniature silicon reed valves or duck bill valves could be used. Reed valves are one way valves that are held shut under pressure. So, the duck bill of each valve is preferably arranged to face into the pouch, or against the flow of fluid from the pouch. If the pouch is squeezed before the detergent delivery system is fitted to the air/water ports of an endoscope, pressure inside the pouch would force the reed valves shut. This, therefore, would prevent detergent solution leaking from the pouch. When the connector is pushed to attach it to the endoscope, the valves are preferably positioned so that the extended air/water ports on the endoscope enter the back of the reed valves and force them open as illustrated in Figure 20. This allows air to enter the pouch and diluted detergent solution to exit the pouch into the endoscope. In the example shown in Figures 17 to 20, the valve 756 is shown fitted only to the liquid outlet port 718, but it will be appreciated that in other examples, a similar or identical valve may also be fitted to the air inlet port.

The present invention therefore provides a detergent delivery system or cleaning apparatus that, in use, is fitted or connected to the air and water ports of an endoscope to permit a detergent solution to be flushed through the air/water channel of the endoscope.

Advantageously, the detergent delivery system comprises a concentrated detergent contained in a reservoir separated from a volume of sterile water prior to use. In this way the quality of the detergent does not deteriorate as quickly as with a pre-mixed low concentration detergent solution. The detergent delivery system further comprises, in preferred embodiments, a feature that only permits air to enter the pouch to pressurise the pouch after the concentrated detergent has been mixed with the sterile water.

The present invention therefore provides an easy to use detergent delivery system that may be connected to an endoscope to provide a detergent solution for cleaning the air/water channel.

## Claims

1. A detergent delivery system (310, 410, 510) comprising:
a flexible bag (312, 412, 512, 612) for holding a liquid;
a first fluid conduit (336, 436, 536, 636) extending between a first end disposed within the internal volume of the bag (312, 412, 512, 612) and a second end (342) disposed external to the bag (312, 412, 512, 612), the second end (342) being connectable to an air port of an endoscope; and
a second fluid conduit (338, 438, 538, 638) extending between a first end disposed within the internal volume of the bag (312, 412, 512, 612) and a second end (346) disposed external to the bag (312, 412, 512, 612), the second end (346) being connectable to a water port of an endoscope,
**characterised in that** a rupturable reservoir (332, 432, 532, 632) for holding a volume of concentrated detergent is disposed within an internal volume of the bag (312, 412, 512, 612) at the first end of the first fluid conduit (336, 436, 536, 636) and the reservoir (332, 432. 532, 632) is rupturable by application of pressure to the reservoir (332, 432, 532, 632) to allow dispensing of the concentrated detergent from the reservoir (332, 432, 532, 632) into the internal volume of the bag (312, 412, 512, 612).

2. A detergent delivery system according to Claim 1, in which each of the first and second fluid conduits (336, 436, 536, 636, 338, 438, 538, 638) extend from a first end of the bag (312, 412, 512, 612), and the first end of the first fluid conduit (336, 436, 536, 636) is at a first distance from a second end of the bag (312, 412, 512, 612) and the first end of the second fluid conduit (338, 438, 538, 638) is at a second distance from the second end of the bag (312, 412, 512, 612), the second distance being different from the first distance.

3. A detergent delivery system according to Claim 1 or Claim 2, in which one or both of the first and second fluid conduits (336, 436, 536, 636, 338, 438, 538, 638) is flexible.

4. A detergent delivery system according to any preceding claim, in which the reservoir (332, 432, 532, 632) comprises a sealed receptacle within the internal volume of the bag (312, 412, 512, 612).

5. A detergent delivery system according to any preceding claim, further comprising a connector (414, 514) attached to the second end of each of the first and second fluid conduits (436, 438, 536, 538), the connector (414, 514) including an air port for connection to an air port of an endoscope, a water port for connection to a water port of an endoscope, a first channel fluidly connecting the first fluid conduit to the air port of the connector (414, 514) and a second channel fluidly connecting the second fluid conduit to the water port of the connector (414, 514).

6. A detergent delivery system according to Claim 5, in which the connector (414, 514) is a rigid element.

7. A detergent delivery system according to Claim 6, in which an axis of each of the air and water ports of the connector (414, 514) is perpendicular to an axis of each of the first and second fluid conduits (436, 438, 536, 538).

8. A detergent delivery system according to any preceding claim, in which the flexible bag (312, 412, 512, 612) contains a first volume of sterile water and the reservoir (332, 432, 532, 632) contains a second volume of detergent, the second volume being less than the first volume.

9. A method of cleaning an air/water channel of an endoscope using a detergent delivery system (310, 410, 510), the detergent delivery system (310, 410, 510) being as claimed in Claim 8, and the method comprising:
fluidly connecting the second end of the first fluid conduit (336, 436, 536, 636) to an air port of the endoscope;
fluidly connecting an end of a second fluid conduit (338, 438, 538, 638) to a water port of the endoscope;
applying pressure to the reservoir to rupture a part of the reservoir (332, 432, 532, 632) to release detergent into the volume of sterile water to form a detergent solution; and
providing a flow of air from the air port, through the first fluid conduit (336, 436, 536, 636) and into the internal volume of the bag (312, 412, 512, 612) to increase pressure in the bag and drive the detergent solution from the bag through the second fluid conduit (338, 438, 538, 638) and through the water port of the endoscope.

10. A method according to Claim 9, in which the reservoir (332, 432, 532, 632) is ruptured by providing a flow of air from the air port and into the first fluid conduit (336, 436, 536, 636) to increase the pressure in the reservoir (332, 432, 532, 632).

## Patentansprüche

1. Reinigungsmittelzuführsystem (310, 410, 510), umfassend:
einen flexiblen Beutel (312, 412, 512, 612) zur Aufnahme einer Flüssigkeit;
eine erste Fluidleitung (336, 436, 536, 636), die sich zwischen einem ersten Ende, das innerhalb des Innenvolumens des Beutels (312, 412, 512, 612) angeordnet ist, und einem zweiten Ende (342) erstreckt,
das außerhalb des Beutels (312, 412, 512, 612) angeordnet ist, wobei das zweite Ende (342) mit einem Luftanschluss eines Endoskops verbindbar ist; und
eine zweite Fluidleitung (338, 438, 538, 638), die sich zwischen einem ersten Ende, das innerhalb des Innenvolumens des Beutels (312, 412, 512, 612) angeordnet ist, und einem zweiten Ende (346) erstreckt,
das außerhalb des Beutels (312, 412, 512, 612) angeordnet ist, wobei das zweite Ende (346) mit einem Wasseranschluss eines Endoskops verbindbar ist,
**dadurch gekennzeichnet,**
**dass** ein aufbrechbarer Vorratsbehälter (332, 432, 532, 632) zur Aufnahme eines Volumens von konzentriertem Reinigungsmittel innerhalb des Innenvolumens des Beutels (312, 412, 512, 612) am ersten Ende der ersten Fluidleitung (336, 436, 536, 636) angeordnet ist und der Vorratsbehälter (332, 432, 532, 632) durch Ausüben von Druck auf den Vorratsbehälter (332, 432, 532, 632) aufbrechbar ist, um die Abgabe des konzentrierten Reinigungsmittels aus dem Vorratsbehälter (332, 432, 532, 632) in das Innenvolumen des Beutels (312, 412, 512, 612) zu ermöglichen.

2. Reinigungsmittelzuführsystem nach Anspruch 1,
bei welchem sich die erste und die zweite Fluidleitung (336, 436, 536, 636, 338, 438, 538, 638) von einem ersten Ende des Beutels (312, 412, 512, 612) aus erstrecken und das erste Ende der ersten Fluidleitung (336, 436, 536, 636) sich in einem ersten Abstand von dem zweiten Ende des Beutels (312, 412, 512, 612) und das erste Ende der zweiten Fluidleitung (338, 438, 538, 638) sich in einem zweiten Abstand vom zweiten Ende des Beutels (312, 412, 512, 612) befindet, wobei der zweite Abstand sich vom ersten Abstand unterscheidet.

3. Reinigungsmittelzuführsystem nach Anspruch 1 oder Anspruch 2,
bei welchem eine oder beide der ersten und zweiten Fluidleitungen (336, 436, 536, 636, 338, 438, 538, 638) flexibel ist/sind.

4. Reinigungsmittelzuführsystem nach einem der vorigen Ansprüche,
bei welchem der Vorratsbehälter (332, 432, 532, 632) einen versiegelten Aufnahmebehälter innerhalb des Innenvolumens des Beutels (312, 412, 512, 612) umfasst.

5. Reinigungsmittelzuführsystem nach einem der vorigen Ansprüche,
welches ferner einen Verbinder (414, 514) umfasst, der an dem zweiten Ende jeder der ersten und zweiten Fluidleitungen (436, 438, 536, 538) angebracht ist, wobei der Verbinder (414, 514) einen Luftanschluss zum Anschließen an einen Luftanschluss eines Endoskops, einen Wasseranschluss zum Anschließen an einen Wasseranschluss eines Endoskops, einen ersten Kanal, der die erste Fluidleitung fluidtechnisch mit dem Luftanschluss des Verbinders (414, 514) verbindet, und einen zweiten Kanal umfasst, der die zweite Fluidleitung fluidtechnisch mit dem Wasseranschluss des Verbinders (414, 514) verbindet.

6. Reinigungsmittelzuführsystem nach Anspruch 5,
bei welchem der Verbinder (414, 514) ein steifes Element ist.

7. Reinigungsmittelzuführsystem nach Anspruch 6,
bei welchem eine Achse sowohl des Luft- als auch des Wasseranschlusses des Verbinders (414, 514) senkrecht zu einer Achse der ersten als auch der zweiten Fluidleitung (436, 438, 536, 538) steht.

8. Reinigungsmittelzuführsystem nach einem der vorigen Ansprüche,
bei welchem der flexible Beutel (312, 412, 512, 612) ein erstes Volumen mit sterilem Wasser und der Vorratsbehälter (332, 432, 532, 632) ein zweites Volumen mit Reinigungsmittel enthält, wobei das zweite Volumen kleiner ist als das erste Volumen.

9. Verfahren zum Reinigen eines Luft- oder Wasserkanals eines Endoskops unter Verwendung eines Reinigungsmittelzuführsystems (310, 410, 510), wobei das Reinigungsmittelzuführsystem (310, 410, 510) nach Anspruch 8 ausgebildet ist und das Verfahren folgendes umfasst:
fluidtechnisches Verbinden des zweiten Endes der ersten Fluidleitung (336, 436, 536, 636) mit einem Luftanschluss des Endoskops;
fluidtechnisches Verbinden eines Endes der zweiten Fluidleitung (338, 438, 538, 638) mit einem Wasseranschluss des Endoskops;
den Vorratsbehälter, um einen Teil des Vorratsbehälters (332, 432, 532, 632) aufzubrechen, um Reinigungsmittel in das Volumen des sterilen Wassers freizusetzen, um eine Reinigungsmittellösung zu bilden; und
Bereitstellen einer Luftströmung aus dem Luftauslass durch die erste Fluidleitung (336, 436, 536, 636) und
in das Innenvolumen des Beutels (312, 412, 512, 612), um den Druck im Beutel zu erhöhen und die Reinigungsmittellösung aus dem Beutel durch die zweite Fluidleitung (338, 438, 538, 638) und durch den Wasseranschluss des Endoskops voranzutreiben.

10. Verfahren nach Anspruch 9,
bei dem der Vorratsbehälter (332, 432, 532, 632) durch Zuführen einer Luftströmung aus dem Luftauslass in die erste Fluidleitung (336, 436, 536, 636) aufgebrochen wird, um den Druck im Vorratsbehälter (332, 432, 532, 632) zu erhöhen.

## Revendications

1. - Système de distribution de détergent (310, 410, 510) comprenant :
un sac souple (312, 412, 512, 612) destiné à contenir un liquide ;
un premier conduit de fluide (336, 436, 536, 636) s'étendant entre une première extrémité disposée dans le volume interne du sac (312, 412, 512, 612) et une seconde extrémité (342) disposée à l'extérieur du sac (312, 412, 512, 612), la seconde extrémité (342) étant apte à être reliée à un orifice d'air d'un endoscope ; et
un second conduit de fluide (338, 438, 538, 638) s'étendant entre une première extrémité disposée dans le volume interne du sac (312, 412, 512, 612) et une seconde extrémité (346) disposée à l'extérieur du sac (312, 412, 512, 612), la seconde extrémité (346) étant apte à être reliée à un orifice d'eau d'un endoscope,
**caractérisé par le fait qu'**un réservoir apte à être rompu (332, 432, 532, 632) destiné à contenir un volume de détergent concentré est disposé dans un volume interne du sac (312, 412, 512, 612) à la première extrémité du premier conduit de fluide (336, 436, 536, 636) et que le réservoir (332, 432, 532, 632) est apte à être rompu par application d'une pression au réservoir (332, 432, 532, 632) pour permettre une distribution du détergent concentré provenant du réservoir (332, 432, 532, 632) dans le volume interne du sac (312, 412, 512, 612).

2. - Système de distribution de détergent selon la revendication 1, dans lequel chacun des premier et second conduits de fluide (336, 436, 536, 636, 338, 438, 538, 638) s'étend à partir d'une première extrémité du sac (312, 412, 512, 612), et la première extrémité du premier conduit de fluide (336, 436, 536, 636) est à une première distance à partir d'une seconde extrémité du sac (312, 412, 512, 612) et la première extrémité du second conduit de fluide (338, 438, 538, 638) est à une seconde distance à partir de la seconde extrémité du sac (312, 412, 512, 612), la seconde distance étant différente de la première distance.

3. - Système de distribution de détergent selon la revendication 1 ou la revendication 2, dans lequel l'un des premier et second conduits de fluide ou les deux (336, 436, 536, 636, 338, 438, 538, 638) est/sont flexible(s).

4. - Système de distribution de détergent selon l'une quelconque des revendications précédentes, dans lequel le réservoir (332, 432, 532, 632) comprend un réceptacle scellé hermétiquement dans le volume interne du sac (312, 412, 512, 612).

5. - Système de distribution de détergent selon l'une quelconque des revendications précédentes, comprenant en outre un raccord (414, 514) attaché à la seconde extrémité de chacun des premier et second conduits de fluide (436, 438, 536, 538), le raccord (414, 514) comprenant un orifice d'air pour une liaison à un orifice d'air d'un endoscope, un orifice d'eau pour une liaison à un orifice d'eau d'un endoscope, un premier canal reliant fluidiquement le premier conduit de fluide à l'orifice d'air du raccord (414, 514) et un second canal reliant fluidiquement le second conduit de fluide à l'orifice d'eau du raccord (414, 514).

6. - Système de distribution de détergent selon la revendication 5, dans lequel le raccord (414, 514) est un élément rigide.

7. - Système de distribution de détergent selon la revendication 6, dans lequel un axe de chacun des orifices d'air et d'eau du raccord (414, 514) est perpendiculaire à un axe de chacun des premier et second conduits de fluide (436, 438, 536, 538).

8. - Système de distribution de détergent selon l'une quelconque des revendications précédentes, dans lequel le sac souple (312, 412, 512, 612) contient un premier volume d'eau stérile et le réservoir (332, 432, 532, 632) contient un second volume de détergent, le second volume étant inférieur au premier volume.

9. - Procédé de nettoyage d'un canal d'air/d'eau d'un endoscope à l'aide d'un système de distribution de détergent (310, 410, 510), le système de distribution de détergent (310, 410, 510) étant tel que revendiqué à la revendication 8, et le procédé comprenant :
raccorder fluidiquement la seconde extrémité du premier conduit de fluide (336, 436, 536, 636) à un orifice d'air de l'endoscope ;
raccorder fluidiquement une extrémité d'un second conduit de fluide (338, 438, 538, 638) à un orifice d'eau de l'endoscope ;
appliquer une pression au réservoir pour rompre une partie du réservoir (332, 432, 532, 632) afin de libérer le détergent dans le volume d'eau stérile pour former une solution de détergent ; et
fournir un écoulement d'air à partir de l'orifice d'air, à travers le premier conduit de fluide (336, 436, 536, 636) et dans le volume interne du sac (312, 412, 512, 612) pour augmenter la pression dans le sac et entraîner la solution de détergent du sac à travers le second conduit de fluide (338, 438, 538, 638) et à travers l'orifice d'eau de l'endoscope.

10. - Procédé selon la revendication 9, dans lequel le réservoir (332, 432, 532, 632) est rompu en fournissant un écoulement d'air à partir de l'orifice d'air et dans le premier conduit de fluide (336, 436, 536, 636) pour augmenter la pression dans le réservoir (332, 432, 532, 632).
